# EUROPEAN PATENT APPLICATION

(11) **EP 1 344 531 A1**
(43) Date of publication of application: **17.09.2003**
(21) Application number: 02005370.8
(22) Date of filing: 15.03.2002
(51) Int. Cl.: A61K 38/04, A61K 38/36, A61P 7/02

(54) **High molecular weight kininogen (HK) domain 5 derived peptides against thrombic diseases**

(71) Applicant: Aventis Behring GmbH, 35002 Marburg (DE)
(72) Inventor: Chavakis, Triantafyllos, Dr., 35392 Giessen (DE); Preissner, Klaus, Prof. Dr., 35394 Giessen (DE)

(57) **Abstract**

Medicaments against thrombotic diseases are described which contain as an active ingredient the domain-5 of high molecular weight kininogen or a domain-5-peptide of the carboxy-terminal region of the high molecular weight kininogen. They are useful as thrombolytic or fibrinolytic agents or as agents against platelet aggregation as well as prophylactic agents against thromboembolic complication.

## Description

Subject of this invention is a medicament against thrombotic diseases which contains as an active ingredient a polypeptide which is derived from the domain-5 of kininogen.

It is known that after vascular injury in patients, pathological thrombosis is the critical event leading to acute myocardial infarction, unstable angina pectoris or stroke. Under physiological conditions, primary haemostasis after vascular injury includes adhesion of platelets to the exposed extracellular matrix in the subendothelium with subsequent platelet aggregation. At high shear rates platelets predominantly adhere to von Willebrand factor and collagen, whereas under low shear or static conditions several other extracellular matrix proteins, including fibrin or vitronectin, mediate initial platelet adhesion via specific interactions between the Arg-Gly-Asp (RGD) sequence of the adhesive matrix proteins and specific adhesion receptors of the αllbβ3-integrin type expressed on platelets.

The temporary platelet thrombus serves as activation surface for the blood coagulation system, culminating in the production of fibrin to seal and stabilize the wound site. A balance between pro-coagulatory and anti-coagulatory factors as well as the contribution of the fibrinolytic system serves to regulate thrombus formation and finally leads to clot dissolution. Tissue-type plasminogen activator (tPA) is the primary protease, which activates plasminogen in a fibrin-specific manner. Subsequently, the formed enzyme plasmin initiates intravascular fibrinolysis. Regulation of plasminogen activation in a fibrin-specific fashion is achieved by plasminogen activator inhibitor-1 (PAI-1), which forms a stable inactive complex with tPA, thereby preventing the initial thrombus against extensive lysis. PAI-1 becomes functionally stabilized by high affinity complex formation with vitronectin, an abundant plasma and wound matrix-associated adhesive glycoprotein, which is also contained together with PAI-1 in platelet α-granules. The binding of PAI-1 to vitronectin occurs primarily within the amino-terminal region of the adhesive protein. This region of vitronectin also contains binding sites for integrins, the urokinase receptor and kininogen. An elevated PAI-1 level constitutes an important thrombotic risk factor for e.g. myocardial infarction or deep venous thrombosis due to an overall increased antifibrinolytic potential.

HK (= high molecular weight kininogen) which is present in plasma at a concentration of 0.67 µM, was initially identified as a non-enzymatic cofactor in the initiation of the contact phase system. HK appears to be associated with vascular injury, inflammation or activation of complement in humoral immune defense. In particular, the enzyme kallikrein liberates the short-lived vasodilator peptide bradykinin from HK domain 4 thereby generating two chain kinin-free HKa. Domain 5 in HKa is rich in His, Gly, and Lys, enabling HKa to bind to anionic surfaces, zinc or heparin. HK/HKa interacts with a number of binding proteins on cell surfaces including the binding protein for globular C1q (gC1qR), urokinase receptor and cytokeratin-1 on endothelial cells, as well as glycoprotein Ib and cell-associated thrombospondin on platelets. It has already been found that HKa and particularly domain 5 bind directly to vitronectin, thereby competing for leukocyte as well as endothelial cell adhesion. Moreover, it has also been previously demonstrated that HKa and PAI-1 compete for proximal binding sites within the amino-terminal domain of vitronectin. Finally, domain 5 of HK binds to the β2-integrin Mac-1 on granulocytes, thereby regulating the Mac-1-dependent leukocyte recruitment in vivo.

Experimental evidence from in vitro and vivo studies renders HK an anti- rather than a prothrombotic agent: A prothrombotic phenotype was reported for kininogen deficient rats, and patients deficient in kininogen or other proteins of the contact phase system are at increased risk for thrombosis:
(i) HK/HKa may regulate pericellular plasmin generation by modulating plasma kallikrein-dependent formation of urokinase plasminogen activator;
(ii) HK domain 3 competes with thrombin for binding to glycoprotein lb and thus inhibits platelet aggregation;

(iii) α-granule-derived HK can inhibit the function of the platelet protease calpain, which plays an important role in mediating integrin-induced signalling and platelet-rich thrombus retraction, and this effect is mainly mediated by domain 2 of HK;
(iv) metabolites of bradykinin block thrombin-induced platelet aggregation.

Based on these in vitro and in vivo properties of kininogen it has been examined whether polypeptides of this endogenous factor are useful in the acute as well as in the prophylactic therapy in vascular diseases and may be valuable as antithrombotic drugs.

It has now been found a medicament against thrombotic diseases which contains as active ingredient the domain-5 of high molecular weight kininogen or a domain-5-peptide of the carboxy-terminal region of the high molecular weight kininogen. Preferred is a medicament wherein the domain-5-peptide is the HK [His-483-Lys502]-peptide whose sequence may be seen from the SEQ ID No.1 of the attached sequence listing. Also, peptides derived from this HK-sequence may be used advantageously if they exhibit antithrombotic efficacy.

Such medicaments for parenteral administration in a therapeutically effective amount, i.e. 0.1-20 mg/kg bodyweight may be used in patients with arterial thrombosis, acute myocardial infarction or lung fibrosis. Moreover, due to their pro-fibrinolytic potential they are valuable to decrease the lysis resistance of unwanted thrombi. Finally, the domain-5-peptides are save and likely non-immunogenic, since they are substances which are derived from endogenous plasma proteins.

The efficacy of domain-5-peptides in said medicaments is based on the scientific finding that another macromolecular ligand for vitronectin, the primary inhibitor of fibrinolysis PAI-1, binds with high affinity to the aminoterminal portion of the adhesive protein within the neighbourhood of binding sites for integrins, urokinase receptor and HK. Thus, PAI-1 does not only constitute another anti-adhesive factor for integrin- and urokinase receptor-mediated cell adhesion to vitronectin, but also competes with activated, two-chain HK (HKa) for interaction with vitronectin. Vice versa, domain 5 of HK and derived polypeptides thereof can efficiently block the binding of PAI-1 to vitronectin or displace the inhibitor from its complex with the adhesive protein, respectively. Since only in complex with vitronectin the PAI-1 function can be stabilized predominantly in the vascular extracellular matrix, dissociation of PAI-1 from vitronectin is associated with the rapid decay of its inhibitor function. Thus, at sites of codistribution of PAI-1 and HKa, the latter factor is able to dissociate the inhibitor from binding to vitronectin and thereby HKa can lower the antifibrinolytic activity of PAI-1. Consequently, HKa and polypeptides thereof may function as a new type of PAI-1 inhibitor and would thus be of therapeutic interest, since increased levels of PAI-1 in patients correlate with an elevated anti-fibrinolytic activity or an increased resistance to thrombolysis. These latter relationships have been evaluated and validated in several clinical studies. Due to the efficiency of kininogen polypeptides to serve as PAI-1 inhibitor, both the anti-fibrinolytic activity as well as the thrombolytic resistance would decrease thereby elevating the net fibrinolytic capacity.

The efficacy of the domain-5-peptide against thrombotic diseases is further increased if they are administered in combination with urokinase or tissue plasminogen activator (tPA) as they eliminate PAI-1. Such a combination medicament is a very powerful agent against thrombotic complications.

The PAI-1 inhibitor activity of kininogen polypeptides may be demonstrated by the following examples:

### Examples of PAI-1 inhibitor activity of kininogen polypeptides

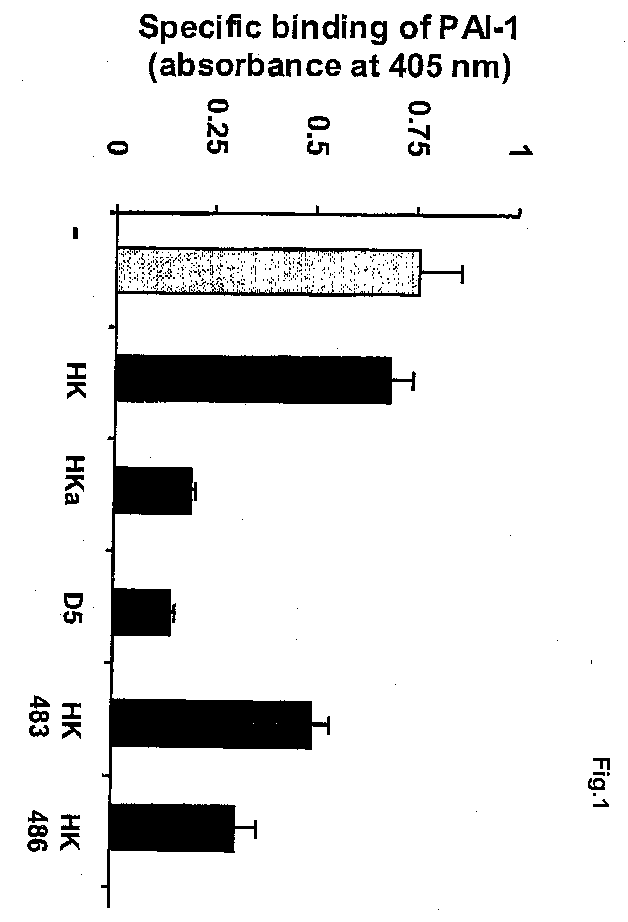

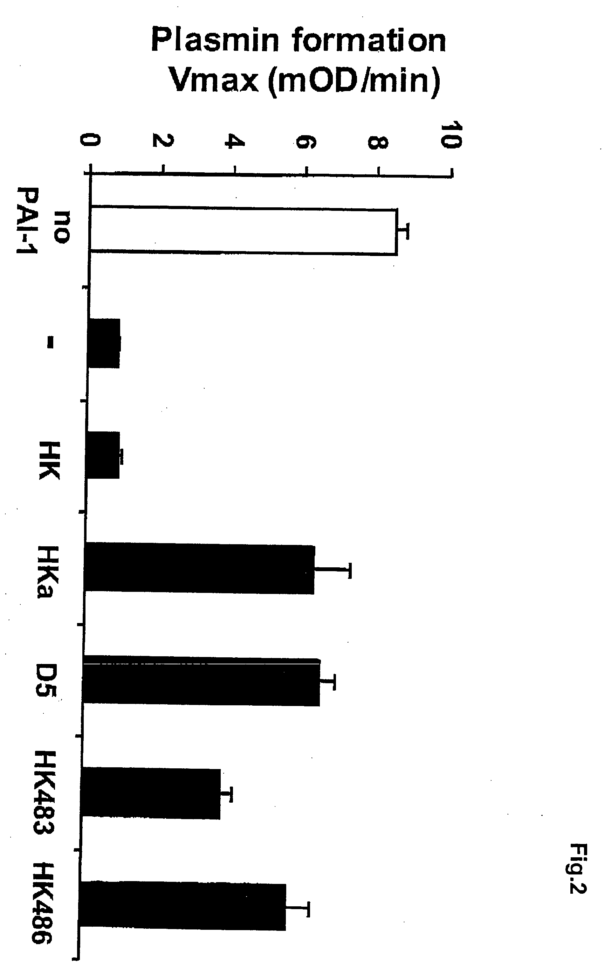

(i) The binding of PAI-1 to vitronectin in PAI-1-depleted (glycine-treated) extracellular matrix of endothelial cells is inhibited by HKa, the isolated domain 5 or the peptides HK483 (His-483 - Gly-497) and HK486 (Gly-486 - Lys-502) derived from the carboxy-terminus of the domain 5 of HK (Fig. 1).
(ii) The antifibrinolytic activity of PAI-1 (inhibition of urokinase-mediated plasminogen activation) is inhibited by the above-mentioned components (Fig. 2).
(iii) HKa, domain 5 or the peptides HK483 and HK486 can thereby enhance urokinase-mediated clot lysis, which is inhibited in the presence of PAI-1 (not shown).

### Binding of PAI-1 to endothelial cell matrix:

The extracellular matrix of human umbilical vein endothelial cells was prepared according to standard procedures. PAI-1-depleted extracellular matrix was obtained by subsequent incubation with glycine (0.1 M, pH 2.3) for 2 h at 25°C. Wells were washed and blocked with phosphate buffered saline containing 3% (wt/vol) bovine serum albumin before proceeding with binding assays. Binding of PAI-1 (2 µg/ml) to the extracellular matrix was performed in a final volume of 50 µl Tris-buffered saline containing 0.1% Tween-20, 0.3% (wt/vol) bovine serum albumin for 2 h at 22°C in the absence or presence of 10 µM ZnCl₂ and different kininogen forms. Following the incubation period and a washing step, bound PAI-1 was quantitated by anti-human PAI-1 monoclonal antibody 12A4, followed by addition of 1:1000 diluted peroxidase-conjugated goat anti-mouse immunoglobulins. The conversion of the substrate 2,2,-azino-di(3-ethyl) benz-thiazoline sulphate was monitored at 405 nm in a Thermomax microtiter plate reader. Nonspecific binding of PAI-1 to bovine serum albumin-coated wells was used as blank and was subtracted to calculate the specific binding.

### Plasminogen activation assays:

PAI-1 (0.1-40 nM) or buffer alone were incubated without or together with multimeric vitronectin (1 µg/ml) in the absence or presence of different kininogen forms and for different time periods (as indicated in the figure legends) in Tris-buffered saline containing 0.1% (wt/vol) Tween-20 and 0.1% (wt/vol) bovine serum albumin at 37°C. After different time intervals, aliquots of reaction mixtures were transferred into microtiter wells and urokinase was added. After an incubation period of 30 min at 22°C, a mixture of plasminogen (final concentration 0.22 µM) and the chromogenic substrate S-2251 (0.8 mM) was added, and the rate of plasmin generation was followed at 405 nm.

In addition to its role as anti-adhesive factor for several cell types, domain 5 of HK was also able to block the adhesion of platelets to vitronectin-rich wound matrix. Moreover, in a concentration-dependent fashion the aggregation of platelet-rich plasma or the formation of a blood platelet fibrin clot, respectively, was blocked in a concentration dependent fashion by HKa, the domain 5 of HK or polypeptides thereof. All these activities of HK are observed within a range of concentrations of the protein or the fragments, respectively, which are within the physiological levels of the plasma protein.

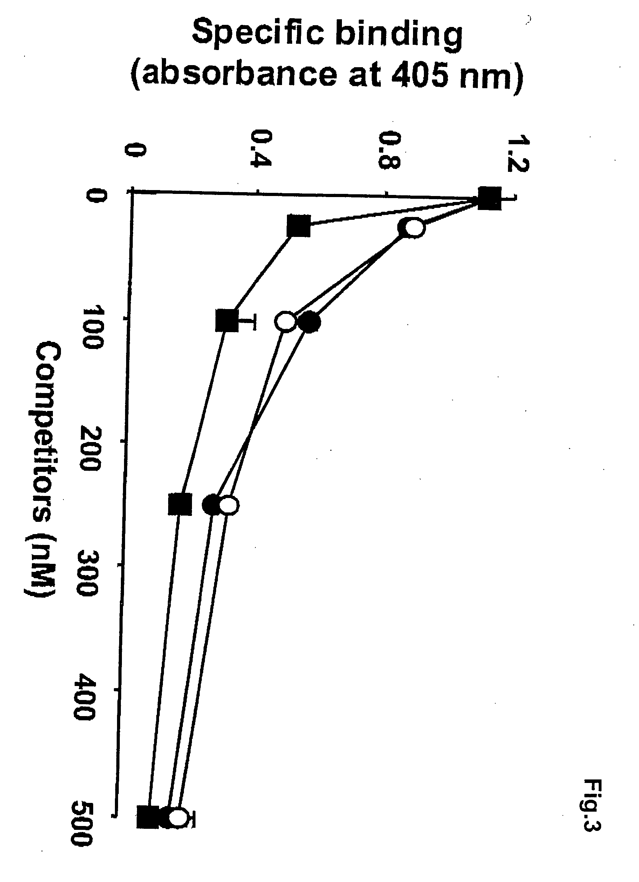

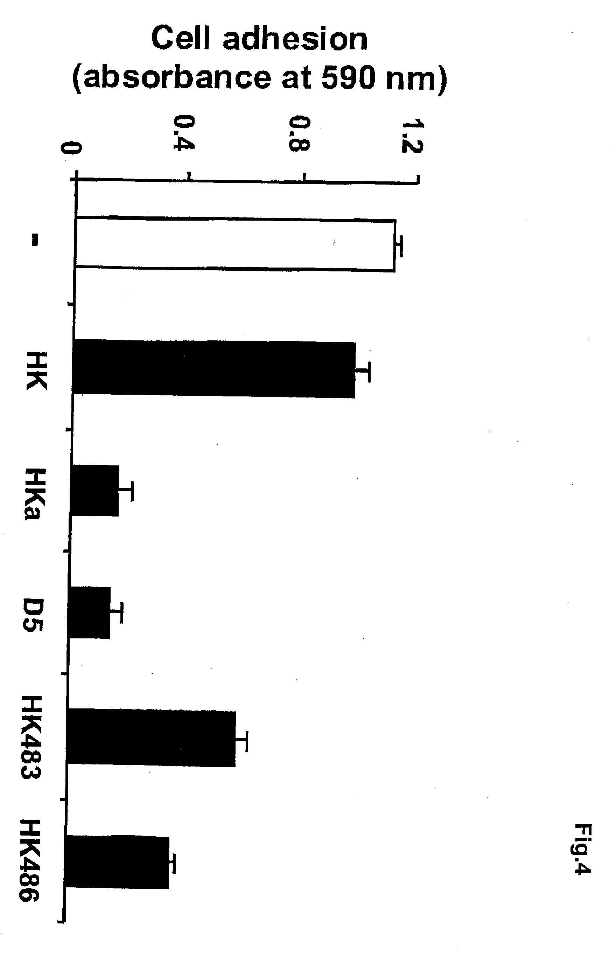

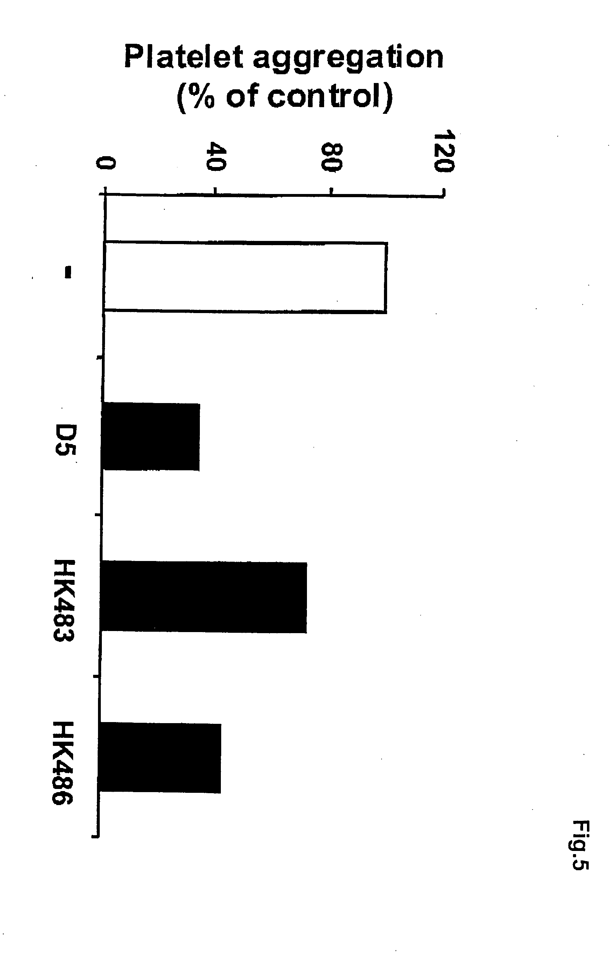

**Examples of anti-platelet activity of kininogen polypeptides**
(i) By binding to vitronectin, HKa, domain 5 or the carboxy-terminal peptides HK483 and HK486 block the interaction of platelet αllbβ3-integrin with vitronectin. This effect is comparable to the anti-adhesive function of PAI-1 (Fig. 3).
(ii) HKa, domain 5 and peptides HK483 and HK486 block αllbβ3-integrin-mediated platelet adhesion to vitronectin (Fig. 4).
(iii) Domain 5 and the peptides HK483 and HK486 inhibit aggregation of platelet-rich plasma (Fig. 5).

### Binding of vitronectin to αllbβ3-integrin:

Microtiter plates were coated with 5 µg/ml αllbβ3-integrin in Tris-buffered saline, pH 7.4, containing 1 mM CaCl₂, 1 mM MgCl₂ overnight at 4°C and then blocked with 3% (w/v) bovine serum albumin. Binding of multimeric vitronectin (2 µg/ml) was performed in a final volume of 50 µl Tris-buffered saline containing 0.1% Tween-20, 0.3% (w/v) bovine serum albumin, 1 mM CaCl₂ and 1 mM MgCl₂ for 2 h at 22°C in the absence or presence of 10 µM ZnCl₂ and different kininogen forms. After the incubation period, monoclonal anti-vitronectin antibody VN7 at a concentration of 125 ng/ml was added, followed by addition of 1:1000 diluted peroxidase-conjugated goat anti-mouse immunoglobulins, respectively. Binding was quantitated as described above.

### Platelet adhesion onto vitronectin-coated plates:

Multiwell plates were coated with 5 µg/ml multimeric vitronectin (dissolved in bicarbonate buffer, pH 9.6) and blocked with 3% (wt/vol) bovine serum albumin for 16 h at 4°C. Platelets stained with calcein acetoxymethyl ester (calcein-AM, 2.5 µM) (15 min at 37°C in the dark) at a density of 5 x 10⁶ cells suspended in 50 µl HEPES-Tyrode buffer containing 4 mM MgCl₂ were added together with the respective competitors and incubated for 30 min at 37°C. After three washes with phosphate buffered saline, bound platelets were lysed with 100 µl of lysis buffer (150 mM NaCI, 10 mM Tris-CHI, pH 7.5, 5mM EDTA, 1% (wt/vol) NP-40, 1 mM phenylmethylsulfonyl fluoride, 20 µg/ml soybean trypsin inhibitor) and quantified in a fluorescent plate reader (excitation filter 492 nm, emission filter 535 nm).

### Aggregation of platelet-rich plasma:

Platelet-rich plasma from fresh whole blood in sodium citrate was prepared by centrifugation of anticoagulated whole blood at 150g for 20 min. After incubation of 0.45 ml of platelet rich plasma with different kininogen forms as indicated in the figure legends, and addition of 5 µl of adenosine-triphosphate at a final concentration of 5 µM, platelet aggregation was induced. Aggregation was quantitated by following the change in light transmission in a standardized aggregometer.
The examples for the treatment with kininogen polypeptides may be summarized as follows:
- Inhibition of platelet aggregation: Prevention of arterial thrombosis leading to acute coronary syndrome, acute myocardial infarction, stroke.
- Inhibition of PAI-1 activity: Prevention of thromboembolic complications (deep venous thrombosis, pulmonary embolism); increased efficiency of lysis of vascular thrombi by plasminogen activators, e.g. during acute therapy of myocardial infarction, stroke or embolic occlusion of a peripheral artery; prevention and therapy of pulmonary fibrosis.

The attached Fig. 1 to Fig. 5 show the following:
- Fig. 1: The binding of 2 µg/ml PAI-1 to PAI-1-depleted (2 h glycine preincubation) extracellular matrix of cultured endothelial cells was tested in the absence (-) or presence of HK, HKa, GST-domain 5 (D5), the peptides HK483 and HK486 (each 500 nM) in a buffer containing 10 µM ZnCl₂. Specific binding of PAI-1 is presented as absorbance at 405 nm. Data are Mean+/-SEM of a typical experiment; similar results were observed in at least 5 separate experiments.
- Fig.2: Urokinase-induced plasmin formation was tested in the presence of 1 µg/ml vitronectin without (open bar) or together with PAI-1 (40 nM) (filled bars) in the absence (-) or presence of HK, HKa, GST-domain 5 (D5), peptide HK483 or peptide HK486 (each 500 nM) in a buffer containing 10 µM ZnCl₂. PAI-1 together with vitronectin in the absence or presence of the different competitors were preincubated for 3 h at 37°C. Urokinase-induced plasmin formation was measured by the addition of a mixture of plasminogen and the chromogenic substrate S-2251. The rate of plasmin formation is expressed as Vmax (mOD/min). Data are Mean+/-SEM of a typical experiment; similar results were observed in at least 5 separate experiments.
- Fig. 3: The binding of vitronectin (2 µg/ml) to immobilized αllbβ3-integrin (5 µg/ml) in the absence or presence of increasing concentrations of PAI-1 (filled squares), HKa (filled circles) or GST-domain 5 (open circles) in a buffer containing 10 µM ZnCl₂ was carried out. Specific binding is presented as absorbance at 405 nm. Data are Mean+/-SEM of a typical experiment; similar results were observed in at least 3 separate experiments.
- Fig. 4.: The adhesion of platelets to vitronectin in the absence (-) or presence of HK, HKa, GST-domain 5(D5), or the peptides HK483 and HK486 (each 250 nM) was carried out. Cell adhesion is presented as % of control. Data are Mean+/-SEM of a typical experiment; similar results were observed in at least 3 separate experiments.
- Fig. 5: Adenosin-diphosphate (5 µM)-induced aggregation of platelet-rich plasma was performed in the absence (-) or presence of GST-domain 5 (D5), or the peptides HK483 and HK486 (each 1000 nM). Platelet aggregation is presented as % of control. Similar results were observed in at least 5 separate experiments.

Sequence Listing

Organization Applicant

Street : Emil-von-Behring-Straße 76
City : Marburg
State :
Country : Germany
PostalCode : 35041
PhoneNumber :
FaxNumber :
EmailAddress :
<110> OrganizationName : Aventis Behring GmbH

Application Project

<120> Title : Medicament against Thrombotic Diseases
<130> AppFileReference : A40
<140> CurrentAppNumber :
<141> CurrentFilingDate :

Sequence

<213> OrganismName : Homo sapiens
<400> PreSequenceString :
HGHGKHKNKG KKNGKHNGWK 20
<212> Type : PRT
<211> Length : 20
SequenceName : A40
SequenceDescription :

## Claims

1. Medicament against thrombotic diseases, **characterized in that** it contains as active ingredient the domain-5 of high molecular weight kininogen or a domain-5-peptide of the carboxy-terminal region of the high molecular weight kininogen.

2. Medicament as claimed in claim 1, **characterized in that** the domain-5-peptide is the HK (His-483 - Lys-502)-peptide or peptides derived from it with antithrombotic efficacy.

3. Medicament as claimed in claims 1 and 2, **characterized in that** it contains the domain-5-peptide in combination with urokinase or tissue plasminogen activator (tPA).

4. Medicament as claimed in claims 1 to 3, **characterized in that** it contains the active ingredients in a preparation suitable for parenteral administration in a therapeutically effective amount.

5. Use of a medicament as claimed in claims 1 to 4, **characterized in that** it is used as thrombolytic or fibrinolytic agent or as an agent against platelet aggregation.

6. Use of a medicament as claimed in claims 1 to 4, **characterized in that** it is used as a prophylactic agent against thromboembolic complications.
